# EUROPEAN PATENT APPLICATION

(11) **EP 4 353 835 A1**
(43) Date of publication of application: **17.04.2024**
(21) Application number: 21955427.6
(22) Date of filing: 31.08.2021
(51) Int. Cl.: C12Q 1/6869, H10N 30/30

(54) **MICROPOROUS STRUCTURE, PREPARATION METHOD, AND CHIP**

(71) Applicant: BGI Shenzhen, Shenzhen, Guangdong 518083 (CN)
(72) Inventor: YUN, Quanxin, Shenzhen, Guangdong 518083 (CN); ZHANG, Yuning, Shenzhen, Guangdong 518083 (CN); LI, Yuxiang, Shenzhen, Guangdong 518083 (CN); ZHANG, Wenwei, Shenzhen, Guangdong 518083 (CN); XU, Xun, Shenzhen, Guangdong 518083 (CN)
(74) Representative: Wilson Gunn
(86) International application number: PCT/CN2021/115777
(87) International publication number: WO 2023/028870

(57) **Abstract**

This application provides a microwell structure, a preparation method and a chip, which belongs to the field of biotechnology. The microwell structure provided in this application comprises a base and support structure layers having through holes and being provided over the base; the support structure layers and the through holes form a cavity with the base; from top to bottom, the support structure layers comprise at least two layers, and the diameter of the through hole in the support structure layer far away from the base is smaller than that of the through hole in the support structure layer provided over the base. With this structure, since the diameter of the through hole in the support structure layer at the top is smaller, it can achieve a better fixing effect on the membrane, thereby increasing the stability of the membrane; the through hole in the support structure layer over the base has a relatively large diameter and communicates with the through hole with a smaller diameter in the support structure layer at the top, thereby obtaining a structure similar to a hole with a smaller opening diameter but a larger volume, which fundamentally solves the stability problem of the membrane and problems of the applicability of a common membrane, the cost and the like , caused by the developing a suitable complex artificial membrane material.

## Description

### TECHNICAL FIELD

This application relates to the field of biotechnology, and in particular to a microwell structure, a preparation method and a chip.

### BACKGROUND

As a commonly used technology in modern molecular biology research, gene sequencing technology has made considerable progress. Current gene sequencing technology comprises the first generation (Sanger) sequencing technology, the Next-generation highthroughput sequencing technology, and the third generation sequencing technology.

The third generation sequencing technology, also known as single molecule gene sequencing technology, can obtain ultra-long direct sequencing data, and can also reduce the difficulty of gene assembly and facilitate the discovery of individualized genetic variation information, which is recognized as one of the advanced sequencing technologies in the future. The principles of the third generation sequencing technology are mainly divided into two camps: optical single molecule sequencing and electrical single molecule sequencing.

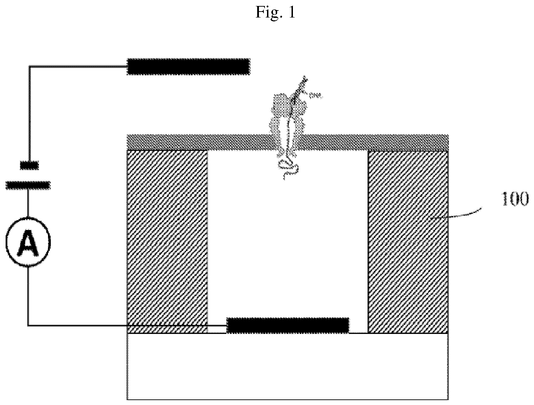

Among them, the representative technology of the electrical single molecule sequencing is the new nanopore sequencing, which is considered by those skilled in the art to be the most promising sequencing solution. Its core sequencing structure and principle are shown in Fig. 1, and the sequencing structure comprises a nanopore protein, a membrane carrying the protein, electrodes applied on both sides of the membrane, and a microwell structure carrying the membrane structure. Due to the different charging properties of the single base A, T, C, or G passing through the microwell, the differences in the electrical signals are collected to realize the sequence analysis.

In this system, the stability of the membrane has a great impact on the quality and data output of the sequencing as well as the use scenarios of the product, and the microwell structure supporting the membrane plays a decisive role in the stability of the membrane. In the prior art, the most common microwell structure is shown in Fig. 1. It is a simple cylindrical structure (100), the effective diameter of the microwell ranges from a few microns to tens of microns to support the membrane (generally being a phospholipid bilayer membrane structure). In practical sequencing applications, in order to increase the data output in a single microwell, it is necessary to increase the diameter of the microwell as much as possible (for example, greater than 100 microns) to increase the reagent capacity within the microwell. However, as the diameter of the microwell increases, the stability of the attached phospholipid membrane will rapidly decrease, thus affecting the entire sequencing process.

For this contradiction, in the prior art, some solutions are given by specially designing the microwell structure, such as increasing the specific surface area of the side wall of the microwell structure, or increasing complex fluid auxiliary structures, or by increasing surface pretreatment and developing a suitable complex artificial membrane material and so on.

However, since the membrane needs to cover the top of the microwell structure, the way based on increasing the specific surface area of the side wall of the microwell structure, whether it is made for the void portion or the solid portion of the microwell structure, still cannot essentially solve the problem of instability of the supported membrane due to its large area; while developing a suitable complex artificial membrane material, reduces the applicability of the membrane (for example, the common low-cost phospholipid membrane material cannot be used), which inevitably increases the cost of sequencing.

In view of these, this application is proposed.

### SUMMARY

This application provides a microwell structure to solve the technical problem in the prior art that the capacity of the microwell (an accommodating space of a reaction system) and the stability of the membrane cannot be taken into account at the same time.

In another aspect, this application also provides a preparation method of a microwell structure, aiming to realize the preparation of the microwell structure. In yet another aspect, this application also provides a sequencing chip to realize the popularization and application thereof.

A microwell structure, comprising a base, and
support structure layers having through holes and being provided over the base;
the support structure layers and the through holes forming a cavity with the base;
from top to bottom, the support structure layers comprise at least two layers, and the diameter or width of the through hole in the support structure layer far away from the base is smaller than the diameter or width of the through hole in the support structure layer provided over the base.

The microwell structure in this application comprises support structure layers provided with through holes (generally being microwells) and a base (generally can contain a necessary electrode); then, the support structure layers, the through holes and the base, by means of their arrangement relationship, together provide a cavity for reaction (i.e., the microwell structure) which is actually an accommodating space of a reaction system. During the specific application process, a membrane over the supporting structure layers is fixed, and substances on the membrane (such as the substance to be measured) pass through the membrane or the channel on the membrane and enter into the cavity. The electrical changes caused by different structures of the substances are also different, so as to realize the analysis of the detection results.

What is particularly critical is that, in this application, from top to bottom, the support structure layers comprise at least two layers, and the diameter or width of the through hole of the support structure layer far away from the base is smaller than the diameter or width of the through hole of the support structure layer provided over the base. With this structure, since the diameter or width of the through hole in the support structure layer at the top is smaller, it can achieve a better fixing effect on the membrane, thereby increasing the stability of the membrane; at the same time, the through hole of the support structure layer over the base has a relatively large diameter or width and communicates with the through hole with a smaller diameter or width of the support structure layer at the top, thereby obtaining a structure similar to a hole with a smaller opening diameter or width but a larger volume, which fundamentally solves the stability problem of the membrane and a series of problems caused by the developing a suitable complex artificial membrane material in the prior art, such as the applicability of a common membrane, the cost and the like.

Preferably, from top to bottom, the support structure layers comprise three layers; the diameter or width of the through hole in the support structure layer at the middle is smaller than the diameter or width of the through hole in the support structure layer at the topmost.

With this design solution, the diameters or widths of the through holes in the support structure layers at the middle and the topmost are both smaller than the diameter or width of the through hole in the support structure layer provided over the base, the three support structure layers can form stepped pores with different diameters or widths, and since the through hole of the support structure layer at the lowest has the largest diameter or width, the volume of the cavity (i.e. the space of the reaction system) is still ensured.

The diameter or width of the through hole in the support structure layer at the middle is smaller than the diameter or width of the through hole in the topmost support structure layer. Therefore, the middle support structure layer also plays a role in fixing and further supporting the topmost support structure layer, thereby further ensuring the stability of the topmost support structure layer and the membrane (which is provided when the reaction is carried out) provided thereon. The above preferred arrangement further structurally ensures the stability of the overall reaction.

Preferably, the projected shape of the through hole comprises one or more of a circular shape, an elliptical shape, or a polygonal shape.

Generally, the most common projected shape of the through hole is a circular shape. On the one hand, it is convenient to set up, and on the other hand, it also ensures the reaction space of the reaction system. It should be understood that, for some special application requirements, the projected shape of the through hole can also be an elliptical shape or a polygonal shape, or even a combination of different shapes.

Preferably, an electrode is provided over the base, and the electrode has a portion exposed to the cavity.

The electrode is a commonly used component for electrochemical reactions to occur. In this solution, preferably, the electrode can serve as a part of the base (provided over the base), and promote the electrochemical reactions of the reaction system in the cavity to occur by being exposed to the cavity.

Preferably, the dimension of the support structure layer provided over the base in a direction perpendicular to the base is larger than those of the support structure layers at the middle and the topmost in the direction perpendicular to the base;
and/or; the dimension of the support structure layer at the middle in the direction perpendicular to the base is less than or equal to the dimension of the support structure layer at the topmost in the direction perpendicular to the base.

In this solution, since the dimension of the support structure layer provided over the base in the direction perpendicular to the base (or can be understood as the thickness of the support structure layer) is the largest, and the diameter or width of its through hole is also the largest, the reaction space within the cavity is ensured, and since its thickness is larger than those of the other two layers, it can provide a better support to the other layers.

In a preferred solution, the diameter or width of the through hole of the middle support structure layer is the smallest, and when looking down from the top of the through holes, the middle support structure layer has a protruding portion, which has a supporting effect on the topmost support structure layer. However, if the thickness thereof is too large, it will affect the stability of the entire layered structure (for example, an invagination may occur), and therefore, it is preferable that the thickness of the support structure layer at the middle is less than or equal to the thickness of the support structure layer at the topmost.

Preferably, the inner wall of the through hole in the support structure layer at the topmost is provided with a plurality of grooves recessed toward the inside of the support structure layer; the grooves extends in a direction from the opening of the through hole of the support structure layer at the topmost to the base.

It should be noted that the extension degree of the groove should not be used as a limitation of this application. The extension degree can be selected according to the actual application situation. For example, in some preferred solutions, the groove can extend throughout the entire inner wall of the through hole of the support structure layer at the topmost. In other embodiments, the groove may only extend partially downward from the opening and may not extend throughout the entire inner wall.

In the above solution, when looking down from the uppermost through hole, the groove structure recessed toward the inside of the topmost support structure layer actually forms a comb teeth-shaped structure, and the specific surface area of the through hole is increased by forming a groove recessed toward the inner wall without enlarging the diameter or width of the through hole, which is beneficial to further enhance the stability of the binding of the membrane and the support structure layers.

The shape of the groove in its depth direction can be any shape, such as a strip shape, or also can be an irregular shape, or the groove also can be a triangular groove, a cylindrical groove, a polygonal groove, etc.

Preferably, along the direction from the opening of the groove to the bottom of the groove, the projected area of the groove in a direction perpendicular to the axial direction of the through hole is gradually increased.

The shape of the above groove forms a structure that gradually expands from the opening to the bottom. Thus, correspondingly, the solid protrusion between each two adjacent grooves and the grooves form similar opposite shapes. This structure is more beneficial to the stability of the support structure and ensures the stability of the membrane and the topmost support structure layer.

In a more preferred solution, when looking down from the through hole, a similar teeth-like structure (i.e., the protrusions between the grooves) is formed on the edge of the inner wall of the through hole in the support structure layer at the topmost. It should be understood that, according to the different application requirements, the planar topology structure of the teeth can be multiple-choice, and the shape of the grooves can actually affect the shape of the formed teeth. The teeth-like structure can be annular comb teeth, triangular comb teeth, or rectangular comb teeth; and it can be a comb teeth shape without sharp corners (such as a rectangular comb teeth), or it also can be a triangular comb teeth with sharp corners.

Preferably, the grooves are evenly provided, and the bottoms and the openings of all the grooves are arc-shaped; the bottom arc sections of all the grooves are collinear to form a circular ring, the opening arc sections of all the grooves are collinear to form another circular ring, and the two circular rings are coaxial.

In the above structure, the grooves are evenly provided and the bottom arc sections and the opening arc sections of all the grooves are collinear to form concentric circular ring structures, so that the layered support structure is more integral, and it is beneficial to the stability of the membrane.

Preferably, the base further comprises a substrate and a dielectric layer; the dielectric layer is provided between the support structure layers and the substrate; when an electrode is provided, the electrode is provided over the substrate, and an adhesion layer is provided between the substrate and the electrode.

Preferably, the dielectric layer has a portion covering the edge of the electrode.

In this solution, the base is further defined and comprises a substrate, a dielectric layer and an optional adhesion layer. Generally, the substrate is provided with a necessary processing circuit for processing signals acquired by the electrode; and the substrate is generally a silicon-based chip or a glass chip prepared by a silicon-based integrated circuit process, or a printed circuit board prepared by a printed circuit board technology.

The adhesion layer can further increase the stability and integrity of the layered structure. In one embodiment, the dielectric layer located between the layered support structure and the substrate can cover all or part of the edge of the electrode.

The material of the support structure layer comprises a silicon-based material, a non-silicon-based material, or a composite material of a silicon-based material and a non-silicon-based material;
and/or;
the electrode is an electrode with electrochemical properties, comprising one of a metal electrode, a silver chloride electrode, an indium tin oxide electrode, a carbon-based material electrode, and a composite electrode composed of carbon-based material and metal;
   and/or;
the material of the dielectric layer comprises a silicon-based material, a non-silicon-based material, or a composite material of a silicon-based material and a non-silicon-based material;
   and/or;
the adhesion layer comprises one or more of titanium, nickel, chromium, titanium nitride materials.

Another aspect of this application provides a preparation method of a microwell structure, which comprises the steps of:
preparing a base; and
forming support structure layers over the base by coating film, photoetching and etching.

Preferably, the support structure layers are prepared in sequence from the one close to the base to the one away from the base;
the coating film comprises one of a physical vapor deposition method, a chemical vapor deposition method, a liquid glue spin coating method, a solid glue film sticking method;
   and/or;
the etching comprises one of a plasma-based dry etching process, a chemical solution-based wet etching process.

Yet another aspect of this application provides a chip, which comprises the above microwell structure.

Preferably, in the chip, over the same base, a plurality of cavities are provided on the support structure layers; and the arrangement of all the cavities comprise a rectangular array arrangement or a rhombus array arrangement.

From the perspective of product structure intensification, integrity and application requirements, there should be a lot of cavity structures formed in the support structure layers, so that the reaction and detection requirements can be met. Therefore, in a preferred solution, a plurality of microwell structures (more specifically, cavities of the support structure layers) can be provided over the same base, and according to the need, the arrangement of the plurality of cavities comprises a rectangular array arrangement or a rhombus array arrangement.

In summary, the microwell structure provided by this application takes into account the reaction space of the system and the stability of the membrane by providing a special cavity structure, and obtains a structure similar to a hole with a smaller opening diameter or width but a larger volume, which fundamentally solves the stability problem of the membrane.

In addition, by means of the specific preferred arrangement of the support structure layers and the selections on the diameter or width of the through hole, the thickness, the shape, etc., the stability of the entire structure is ensured, which further provides a guarantee for the reaction system.

In addition, by forming grooves with specific shapes in the inner wall of the support structure layer at the topmost, the specific surface area of the inner wall increases, which is beneficial to further enhance the stability of the binding of the membrane and the support structure layers.

### BRIEF DESCRIPTION OF DRAWINGS

To describe the technical solutions in the embodiments of this application or in the prior art more clearly, the following will briefly describe the drawings required for describing the embodiments or the prior art. Apparently, the drawings described below are merely some embodiments of this application, and ordinary persons skilled in the art may still derive other drawings from these drawings without creative efforts.
Fig. 1 is a schematic view of a microwell sequencing structure in the prior art;
Fig. 2a is a schematic cross-sectional view of a microwell structure according to an embodiment of this application comprising three support structure layers;
Fig. 2b is a schematic top plan view of a microwell structure according to an embodiment of this application comprising three support structure layers;
Fig. 3a is a schematic top plan view of a microwell structure according to another embodiment of this application comprising three support structure layers;
Fig. 3b is a schematic top plan view of a microwell structure according to yet another embodiment of this application comprising three support structure layers;
Fig. 4a is a schematic view of a chip according to an embodiment of this application, with the arrangement of the cavities being a rectangular array arrangement;
Fig. 4b is a schematic view of a chip according to another embodiment of this application, with the arrangement of the cavities being a rhombus array arrangement;
Fig. 5a, Fig. 5b and Fig. 5c are respectively schematic views of different preparation stages of a preparation method of a microwell structure according to an embodiment;
Fig. 6 is a schematic view of a base according to an embodiment of this application.

### Reference signs:

100. Cylindrical structure;
201. Base; 202. Support structure layers; 203. Cavity;
2011. Electrode; 2012. Substrate; 2013. Dielectric layer;
2021. First structure layer; 2022. Second structure layer; 2023. Third structure layer;
2031. First microwell; 2032. Second microwell; 2033. Third microwell;
301. Groove; 302. First circular ring; 303. Second circular ring.

### DESCRIPTION OF EMBODIMENTS

In order to make the above and other features and advantages of this application clearer, this application is further described below in conjunction with the drawings. It should be understood that the specific embodiments given herein are for the purpose of explanation to those skilled in the art, only illustrative and not restrictive.

In the description of this application, it is to be understood that orientation or position relationships indicated by terms "center", "longitudinal", "transverse", "length", "width", "thickness", "upper", "lower", "front", "rear", "left", "right", "vertical", "horizontal", "top", "bottom", "inner", "outer", "clockwise", "counterclockwise" are orientation or position relationships shown on the basis of the drawings, and it is not intended to indicate or imply that the referred device or element must be at the specific orientation or be constructed and operated at the specific orientation, but only intended for the convenience of describing this application and simplifying the description, and thus such orientation or position relationships may not be understood as limits to this application.

In addition, terms "first" and "second" are only adopted for the objective of description, and may not be understood to indicate or imply relative importance of the indicated technical feature or implicitly indicate the number of the indicated technical feature. Therefore, a feature limited by "first" or "second" may explicitly or implicitly comprise at least one such feature. In the description of this application, unless otherwise explicitly and specifically limited, "a plurality of" means at least two, such as two, three, etc.

In this application, unless otherwise explicitly specified and limited, terms such as "mount", "connect", "connection" and "fix" should be broadly understood, for example: they may refer to a fixed connection, and may also refer to a detachable connection or being integrated; they may refer to a mechanical connection or may also refer to an electrical connection; they may refer to a direct connection, or may also refer to an indirect connection through an intermediate; they may refer to communication inside two elements or interaction relationship of two elements, unless otherwise explicitly specified and limited. For those of ordinary skill in the art, the specific meanings of the above terms in this application may be understood according to actual conditions.

In this application, unless otherwise explicitly specified and limited, a first feature is "over" or "under" a second feature, which can be a direct contact between the first and second features, or an indirect contact between the first and second features through an intermediate. Moreover, a first feature is "over", "above" or "on top of" a second feature, which can be that the first feature is directly above or obliquely above the second feature, or only represents that the horizontal height of the first feature is larger than that of the second feature. A first feature is "under", "below" or "underneath" a second feature, which can be that the first feature is directly below or obliquely below the second feature, or only represents that the horizontal height of the first feature is smaller than that of the second feature.

In the description of this specification, the description with reference to the terms "one embodiment", "some embodiments", "example", "specific example", or "some examples" or the like means that a particular feature, structure, material, or characteristic described in connection with the embodiment or example is comprised in at least one embodiment or example of this application. In this specification, exemplary description of the above terms is not necessarily referring to the same embodiment or example. Furthermore, the particular features, structures, materials, or characteristics described may be combined in a suitable manner in any one or more embodiments or examples. In addition, those skilled in the art may combine and incorporate different embodiments/examples or features of different embodiments/examples described in this specification unless they are inconsistent with each other.

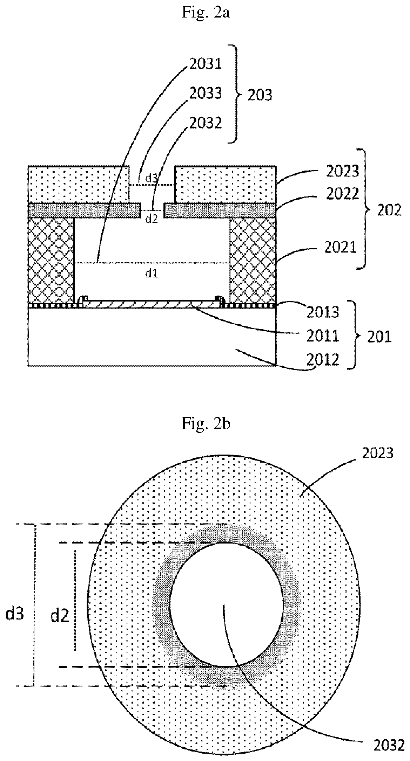

Referring to Figs. 2a to 6, in one embodiment of this application, a microwell structure is provided, which comprises a base 201, and support structure layers 202 having through holes and being provided over the base 201; the support structure layers 202 and the through holes form a cavity 203 with the base 201; from top to bottom, the support structure layers 202 comprise at least two support structure layers, and the diameter or width of the through hole in the support structure layer 202 far away from the base 201 is smaller than the diameter or width of the through hole in the support structure layer 202 provided over the base 201.

In some further embodiments, other embodiments of this application can also be further definitions or additions based on the above embodiment, i.e., one of the following solutions or a combination of a plurality of solutions of the following solutions. For example, from top to bottom, the support structure layers 202 comprise three layers; the diameter or width of the through hole in the support structure layer 202 at the middle is smaller than the diameter or width of the through hole in the support structure layer 202 at the topmost. Alternatively, the projected shape of the through hole comprises one or more of a circular shape, an elliptical shape, a polygonal shape. Alternatively, an electrode 2011 is provided over the base 201, and the electrode 2011 has a portion exposed to the cavity 203. Alternatively, the dimension of the support structure layer 202 provided over the base 201 in a direction perpendicular to the base 201 is larger than the dimensions of the support structure layers 202 at the middle and the topmost in the direction perpendicular to the base 201. Alternatively, the dimension of the support structure layer 202 at the middle in the direction perpendicular to the base 201 is less than or equal to the dimension of the support structure layer 202 at the topmost in the direction perpendicular to the base 201. Alternatively, the inner wall of the through hole in the support structure layer 202 at the topmost is provided with a plurality of grooves 301 recessed toward the inside of the support structure layer 202; the grooves 301 extends in a direction from the opening of the through hole of the support structure layer 202 at the topmost to the base 201. Alternatively, along the direction from the opening of the groove 301 to the bottom of the groove 301, the projected area of the groove 301 in a direction perpendicular to the axial direction of the through hole is gradually increased. Alternatively, the grooves 301 are evenly provided, and the bottoms and the openings of all the grooves 301 are arc-shaped; the bottom arc sections of all the grooves 301 are collinear to form a circular ring, the opening arc sections of all the grooves 301 are collinear to form another circular ring, and the two circular rings are coaxial. Alternatively, the base 201 further comprises a substrate 2012 and a dielectric layer 2013; the dielectric layer 2013 is provided between the support structure layers 202 and the substrate 2012; when an electrode 2011 is provided, the electrode 2011 is provided over the substrate 2012, and an adhesion layer is provided between the substrate 2012 and the electrode 2011. Alternatively, the dielectric layer 2013 has a portion covering the edge of the electrode 2011. Alternatively, the material of the support structure layer 202 comprises a silicon-based material, a non-silicon-based material, or a composite material of a silicon-based material and a non-silicon-based material. Alternatively, the electrode 2011 is an electrode 2011 with electrochemical properties, comprising one of a platinum electrode, a gold electrode, a palladium electrode, a silver electrode, a silver chloride electrode, an indium tin oxide electrode, a carbon nanotube electrode, a graphene electrode, a composite electrode composed of carbon nanotube, graphene and metal. Alternatively, the material of the dielectric layer 2013 comprises a silicon-based material, a non-silicon-based material, or a composite materials of a silicon-based material and a non-silicon-based material. Alternatively, the adhesion layer comprises one or more of titanium, nickel, chromium, titanium nitride materials.

In one specific embodiment of this application, the microwell structure comprises a base 201 and support structure layers 202; an electrode 2011 (having a portion exposed to a cavity 203) is provided over the base 201, through holes are provided in the support structure layers 202, and the support structure layers 202 and the through holes form the cavity 203 with the base 201; from top to bottom, support structure layers 202 comprise two layers, and the diameter or width of the through hole in the support structure layer 202 far away from the base 201 is smaller than the diameter or width of the through hole in the support structure layer 202 provided over the base 201.

In some embodiments, the support structure layers 202 comprise three layers; and the diameter or width of the through hole in the support structure layer 202 at the middle is smaller than the diameter or width of the through hole in the support structure layer 202 at the topmost. The base 201 comprises a substrate 2012, an electrode 2011 and a necessary dielectric layer 2013; the dielectric layer 2013 is provided between the support structure layers 202 and the substrate 2012; the electrode 2011 is provided over the substrate 2012, and an adhesion layer is provided between the substrate 2012 and the electrode 2011.

As shown in Fig. 2a, for the convenience of understanding, in the specific embodiment in which the support structure layers 202 comprise three layers, the support structure layers 202, from bottom to top, are sequentially referred to as a first structure layer 2021, a second structure layer 2022, and a third structure layer 2023. Correspondingly, the through holes formed in each structure layer are referred to as a first microwell 2031, a second microwell 2032 and a third microwell 2033.

More specifically, for example, in the three-levels through holes formed from top to bottom:
the diameter (d3) or width of the third microwell 2033 is between 1 micron and 200 microns, and specifically can be 50 microns;
the diameter (d2) or width of the second microwell 2032 is between 1 micron and 200 microns, and specifically can be 30 microns;
the diameter (d1) or width of the first microwell 2031 is between 10 microns and 10 millimeters, and specifically can be 100 microns.

In terms of material selection, in some embodiments, the material of the support structure layer 202 comprises a cross-linkable organic polymer material, such as SU8, PDMS and similar organic photoresist materials; a wet glue adopted by a spin coating process or a dry glue adopted by a film sticking process can also be used, and the dry glue is preferred, which is beneficial to ensure the uniformity and consistency of the thickness.

In addition to the above materials, the support structure layer 202 can also be a silicon-based medium, such as silicon oxide, silicon nitride, silicon oxynitride, etc., or a non-silicon-based medium, such as photoresist and other organics, or a composite material of a silicon-based medium and a non-silicon-based medium .

The electrode 2011 is an electrode 2011 with electrochemical properties, comprising one of a platinum electrode 2011, a gold electrode 2011, a palladium electrode 2011, a graphene electrode 2011 (the platinum electrode 2011 is the most preferred). In some other embodiments, the electrode 2011 can also be a silver electrode or a silver chloride electrode, or can also be an indium tin oxide electrode, or can also be a carbon nanotube electrode or a graphene electrode, or can also be a composite electrode 2011 of metal (such as platinum, gold, palladium, silver, etc.) and carbon nanotube or graphene.

The substrate 2012 can be a glass wafer, a silicon wafer, an integrated circuit board, or a printed circuit board, etc. The dielectric layer 2013 can be a non-silicon-based medium, or a composite medium of a silicon-based medium and a non-silicon-based medium, such as SU8 (SU8 photoresist), PDMS (polydimethylsiloxane) and other organic polymer materials. The adhesion layer is titanium or nickel metal material.

It should be noted that, in some embodiments, for the support structure layers 202, the first structure layer 2021, the second structure layer 2022 and the third structure layer 2023 can be made of the same material. In other embodiments, the first structure layer 2021, the second structure layer 2022 and the third structure layer 2023 can be made of different materials.

In some preferred embodiments, the projected shape (the planar topology structure) of the through hole can be one or a combination of multiple shapes of a circular shape, an elliptical shape, a polygonal shape, and the circular shape is the most preferred.

In some of the embodiments, the dimension of the support structure layer 202 over the base 201 in a direction perpendicular to the base 201 is larger than the dimensions of the support structure layers 202 at the middle and the topmost in the direction perpendicular to the base 201; the dimension of the support structure layer 202 at the middle in the direction perpendicular to the base 201 is less than or equal to the dimension of the support structure layer 202 at the topmost in the direction perpendicular to the base 201.

More specifically, for example, in the three-level support structure layers 202 formed from top to bottom:
the thickness of the third structure layer 2023 can be 100 nanometers to 10 millimeters, such as 50 microns;
the thickness of the second structure layer 2022 can be 100 nanometers to 10 millimeters, such as 50 microns;
the thickness of the first structure layer 2021 can be between 1 micron and 10 millimeters, such as 100 microns.

The first structure layer 2021 provided over the base 201 has the largest thickness, and the diameter or width of its through hole is also the largest, ensuring a reaction space within the cavity 203, and since its thickness is larger than those of the other two layers, it can provide a better support to the other structure layers. The thickness of the second structure layer 2022 and the diameter or width of its through hole are the smallest, and when looking down from the top of the through holes, the second structure layer 2022 has a protruding portion, which has a supporting effect on the third structure layer 2023. However, if the thickness thereof is too large, it will affect the stability of the entire layered structure (for example, an invagination may occur), and therefore, it is preferable that the thickness of the second structure layer 2022 is less than or equal to the thickness of the third structure layer 2023.

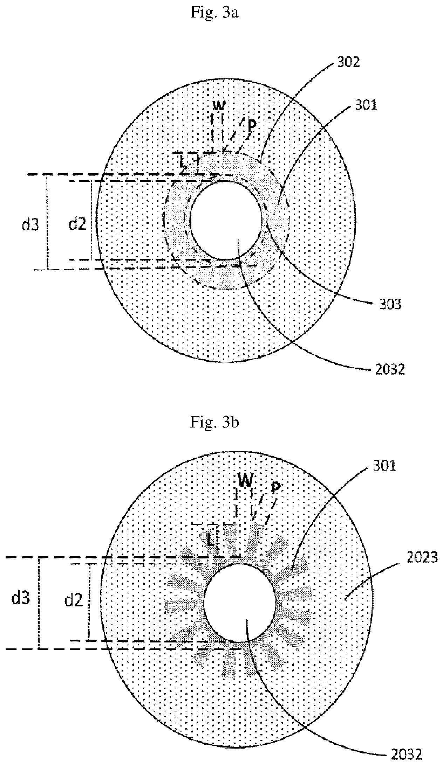

Referring to Figs. 3a and 3b, in some other preferred solutions, the inner wall of the third microwell 2033 of the third structure layer 2023 is provided with a plurality of grooves 301 recessed toward the inside of the third structure layer 2023; the grooves 301 extends in a direction from the opening of the third microwell 2033 to the base 201. At the same time, along the direction from the opening of the groove 301 to the bottom of the groove 301, in a further solution, referring to Fig. 3b, the projected area of the groove 301 in a direction perpendicular to the axial direction of the through hole is gradually increased, while in Fig. 3a, the projected area of the groove 301 in a direction perpendicular to the axial direction of the through hole shows a gradually decreasing trend or very weak change trend. It should be understood that the changes of the projected area of the groove 301 in a direction perpendicular to the axial direction of the through hole are only some preferred solutions in the various embodiments of this application, and are not intended to limit the protection scope of this application.

In the above solution, when looking down from the third microwell 2033, such structure of grooves 301 actually forms a comb teeth-shaped structure, and the specific surface area of the third microwell 2033 is increased by forming grooves 301 recessed toward the inner wall without enlarging the diameter or width of the through hole, which is beneficial to further enhance the stability of the binding of the membrane (which is covered on the third microwell 2033 during application) and the third structure layer 2023.

In addition, the shape of the groove 301 forms a structure that gradually expands from the opening to the bottom. Thus, correspondingly, the solid protrusion between each two adjacent grooves 301 and the grooves form similar opposite shapes. This structure is more stable and can ensure the stability of the membrane and the topmost support structure layer 202.

In some other embodiments, the grooves 301 are evenly provided, and the bottoms and the openings of all the grooves 301 are arc-shaped; the bottom arc sections of all the grooves 301 are collinear to form a circular ring, also known as a first circular ring 302 (refer to Fig. 3a, which is actually a discontinuous circular ring); the opening arc sections of all the grooves 301 are collinear to form another circular ring, also known as a second circular ring 303 (refer to Fig. 3a, which is actually a discontinuous circular ring), and the first circular ring 302 and the second circular ring 303 are coaxial.

The grooves 301 are evenly provided, and the bottom arc sections and the opening arc sections of all the grooves 301 are collinear to form concentric circular ring structures, so that the layered support structure is more integral, and it is beneficial to the stability of the membrane. More preferably, the first microwell 2031, the second microwell 2032 and the third microwell 2033 are coaxially provided.

More specifically, the distance between the bottommost parts of each two adjacent grooves 301 is denoted by W, the distance from the opening to the bottom of the groove 301 is denoted by L, the distance between the two endpoints of the arc at the bottom of the groove 301 is denoted by P. For the sake of structural stability, optimization of specific surface area and stable support for the membrane, L is between 1 micron and 5 millimeters, such as 30 microns; P is between 1 micron and 100 microns, such as 5 microns; W is between 1 micron and 100 microns, such as 5 microns.

It should be understood that the saw-teeth structure containing the grooves 301 shown in Figs.3a and 3b is actually a hollow structure composed of the third microwell 2033 and the grooves 301.

In one embodiment of this application, a preparation method of a microwell structure is provided, which comprises the steps: preparing a base 201; and forming support structure layers 202 over the base 201 by coating film, photoetching and etching.

In a further preferred method, it comprises the following steps:
101: preparing a base 201;
in this step, firstly, a base 201 is provided, which mainly comprises a necessary substrate 2012, an electrode 2011 and a dielectric layer 2013, etc.; the method for preparing the base 201 is generally well-known in the art and will not be described in detail in this application;
102: preparing a first structure layer 2021, a second structure layer 2022 and a third structure layer 2023 over the base 201 in sequence by coating film, photoetching and etching.

The coating film comprises one of a physical vapor deposition method, a chemical vapor deposition method, a liquid glue spin coating method, a solid glue film sticking method; the etching comprises one of a plasma-based dry etching process, a chemical solution-based wet etching process.

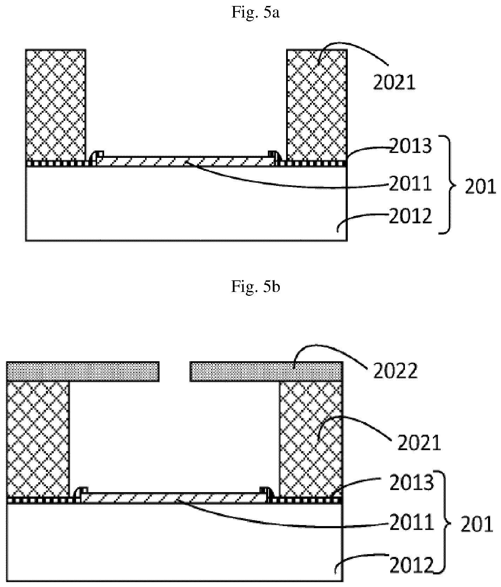

In this step, specifically, the first structure layer 2021 is prepared over the substrate 2012 by coating film, photoetching and etching, as shown in Fig. 5a. The coating film for the first structure layer 2021 may comprise a physical vapor deposition method, a chemical vapor deposition method, a liquid glue spin coating method, a solid glue film sticking method, etc. In order to ensure the uniformity and consistency of the thickness of the first structure layer 2021, it is preferable to use a solid glue film sticking method, and the solid glue film is preferably a cross-linkable polymer photoresist, such as SU8. The etching for the first structure layer 2021 may be a plasma-based dry etching process, or also may be a chemical solution-based wet etching process, and preferably, a developer-based chemical development etching process is used.

The second structure layer 2022 is further prepared by coating film, photoetching and etching, as shown in Fig. 5b. The coating film for the second structure layer 2022 usually comprise a physical vapor deposition method, a chemical vapor deposition method, a liquid glue spin coating method, a solid glue film sticking method, etc. In order to ensure the uniformity and consistency of the thickness of the first structure layer 2021, it is preferable to use a solid glue film sticking method, and the solid glue film is preferably a cross-linkable polymer photoresist, such as SU8. The etching for the second structure layer 2022 may be a plasma-based dry etching process, or also may be a chemical solution-based wet etching process, and preferably, a developer-based chemical development etching process is used.

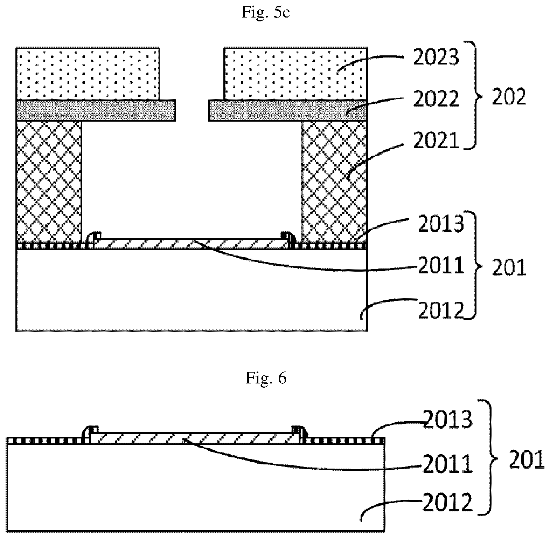

The third structure layer 2023 is further prepared by coating film, photoetching and etching, as shown in Fig. 5c. The coating film for the third structure layer 2023 usually comprise a physical vapor deposition method, a chemical vapor deposition method, a liquid glue spin coating method, a solid glue film sticking method, etc. In order to ensure the uniformity and consistency of the thickness of the third structure layer 2023, it is preferable to use a solid glue film sticking method, and the solid glue film is preferably a cross-linkable polymer photoresist, such as SU8. The etching for the third structure layer 2023 may be a plasma-based dry etching process, or also may be a chemical solution-based wet etching process, and preferably, a developer-based chemical development etching process is used.

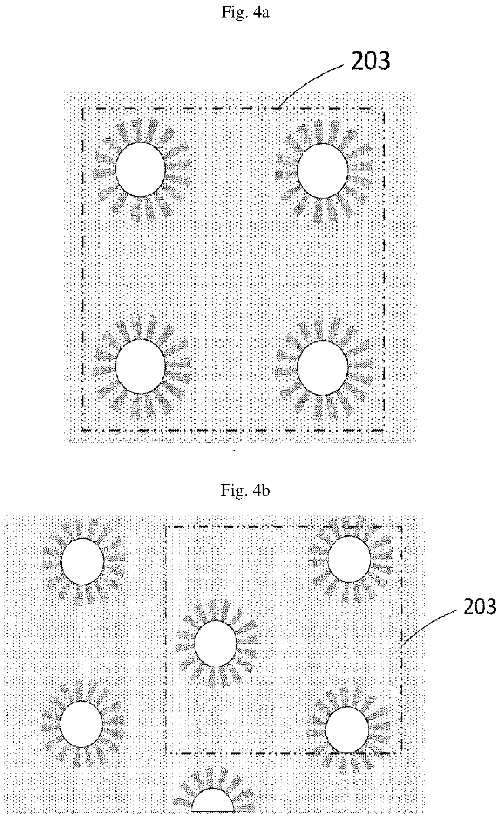

Referring to Figs. 4a and 4b, in one embodiment of this application, a chip containing a microwell structure is provided. In a preferred solution, over the same base 201, a plurality of cavities 203 are provided on the support structure layers 202 , the plurality of cavities 203 being in a periodic arrangement; and the arrangement of all the cavities 203 comprises a rectangular array arrangement or a rhombus array arrangement. In Figs. 4a and 4b, there is no fundamental difference in the two arrangements of cavities 203, and a different array arrangement of the microwell structures is selected mainly based on the selected base 201. In Figs. 4a and 4b, it should be understood that, as can be seen from the shape of the cavity 203 in top plan view, it is actually shown that, in the support structure layers 202, the diameter (d3) or width of the third microwell 2033 is the same as the diameter (d2) or width of the second microwell 2032, wherein the hollow circle in the center actually represents the diameter (d3) or width of the third microwell 2033 and the diameter (d2) or width of the second microwell 2032, and the gray parts surrounding the center of the circle are actually hollow grooves 301.

Although the embodiments of this application have been shown and described above, it can be understood that the above embodiments are illustrative and should not be construed as limitations of this application, and ordinary persons skilled in the art can make changes, modifications, substitutions and variations to the above embodiments without departing from scope of this application.

## Claims

1. A microwell structure, comprising a base, and
support structure layers having through holes and being provided over the base;
wherein the support structure layers and the through holes form a cavity with the base;
wherein from top to bottom, the support structure layers comprise at least two layers, and the diameter or width of the through hole in the support structure layer far away from the base is smaller than the diameter or width of the through hole in the support structure layer provided over the base.

2. The microwell structure according to claim 1, wherein, from top to bottom, the support structure layers comprise three layers;
the diameter or width of the through hole in the support structure layer at the middle is smaller than or equal to the diameter or width of the through hole in the support structure layer at the topmost.

3. The microwell structure according to claim 1, wherein the projected shape of the through hole comprises one or more of a circular shape, an elliptical shape, a polygonal shape.

4. The microwell structure according to claim 1, wherein an electrode is provided over the base, and the electrode has a portion exposed to the cavity.

5. The microwell structure according to claim 2, wherein the dimension of the support structure layer provided over the base in a direction perpendicular to the base is larger than the dimensions of the support structure layers at the middle and the topmost in the direction perpendicular to the base;
and/or;
the dimension of the support structure layer at the middle in the direction perpendicular to the base is less than or equal to the dimension of the support structure layer at the topmost in the direction perpendicular to the base.

6. The microwell structure according to claim 2, wherein the inner wall of the through hole in the support structure layer at the topmost is provided with a plurality of grooves recessed toward the inside;
the grooves extends in a direction from the opening of the through hole of the support structure layer at the topmost to the base.

7. The microwell structure according to claim 6, wherein, along the direction from the opening of the groove to the bottom of the groove, the projected area of the groove in a direction perpendicular to the axial direction of the through hole is gradually increased.

8. The microwell structure according to claim 6, wherein the grooves are evenly provided, and the bottoms and the openings of all the grooves are arc-shaped;
the bottom arc sections of all the grooves are collinear to form a circular ring, the opening arc sections of all the grooves are collinear to form another circular ring, and the two circular rings are coaxial.

9. The microwell structure according to any one of claims 1 to 8, wherein the base further comprises a substrate and a dielectric layer;
the dielectric layer is provided between the support structure layers and the substrate;
when an electrode is provided, the electrode is provided over the substrate, and an adhesion layer is provided between the substrate and the electrode.

10. The microwell structure according to claim 9, wherein the dielectric layer has a portion covering the edge of the electrode.

11. The microwell structure according to claim 9, wherein the material of the support structure layer comprises a silicon-based material, a non-silicon-based material, or a composite material of a silicon-based material and a non-silicon-based material;
and/or;
the electrode is an electrode with electrochemical properties, comprising one of a metal electrode, a silver chloride electrode, an indium tin oxide electrode, a carbon-based material electrode, a composite electrode composed of carbon-based material and metal;
and/or;
the material of the dielectric layer comprises a silicon-based material, a non-silicon-based material, or a composite material of a silicon-based material and a non-silicon-based material;
and/or;
the adhesion layer comprises one or more of titanium, nickel, chromium, titanium nitride materials.

12. A preparation method of a microwell structure, comprising the steps of:
providing a base;
forming support structure layers over the base by coating film, photoetching and etching.

13. The preparation method of a microwell structure according to claim 12, wherein the support structure layers are prepared in sequence from the one close to the base to the one away from the base;
the coating film comprises one of a physical vapor deposition method, a chemical vapor deposition method, a liquid glue spin coating method, a solid glue film sticking method;
and/or;
the etching comprises one of a plasma-based dry etching process, a chemical solution-based wet etching process.

14. A chip, comprising the microwell structure according to any one of claims 1-11.

15. The chip according to claim 14, wherein, over the same base, a plurality of cavities are provided on the support structure layers;
and the arrangement of all the cavities comprise a rectangular array arrangement or a rhombus array arrangement.
